# EUROPÄISCHE PATENTSCHRIFT

(11) **EP 0 893 134 B1**
(45) Veröffentlichungstag und Bekanntmachung des Hinweises auf die Patenterteilung: **06.05.2004**
(21) Anmeldenummer: 98112999.2
(22) Anmeldetag: 13.07.1998
(51) Int. Cl.: A61M 5/44

(54) **Vorrichtung zum Auftauen und/oder zum Erwärmen von in einem Behältnis befindlichem medizinischem Infusions- oder Transfusionsgut**
Device for thawing and/or warming of medical infusion or transfusion material in a container
Dispositif pour décongeler et/ou réchauffer une matière d'infusion ou de transfusion située dans un récipient

(30) Priorität: 18.07.1997 DE 19730994
(43) Veröffentlichungstag der Anmeldung: 27.01.1999
(73) Patentinhaber: TRANSMED Medizintechnik GmbH, 33181 Wünnenberg (DE)
(72) Erfinder: Pelster, Michael, 48231 Warendorf (DE)

(56) Entgegenhaltungen:
- WO-A1-88/09186
- DE-A1- 3 047 784

## Beschreibung

Die vorliegende Erfindung betrifft allgemein das Gebiet der medizintechnischen Geräte und insbesondere eine Vorrichtung zum Auftauen und/oder zum Erwärmen von in einem Behältnis befindlichem medizinischem Infusions- oder Transfusionsgut, beispielsweise eines Blutpräparats wie etwa einer Blutkonserve oder einer Blutplasmakonserve.

Derartiges Infusions- oder Transfusionsgut wird im Regelfall im tiefgefrorenen oder gekühlten Zustand gelagert, so daß dessen Temperatur während der Aufbewahrung und Bereithaltung weit unterhalb der Körpertemperatur liegt. Um beispielsweise bei einer Operation oder in einem Notfall zur Verfügung zu stehen, muß das tiefgefrorene bzw. gekühlte Infusions- oder Transfusionsgut vor der Retransfusion schnell und gleichwohl schonend aufgetaut bzw. erwärmt werden, um die Empfängerin bzw. den Empfänger des Infusions- oder Transfusionsguts vor Unterkühlungen und den daraus resultierenden, zum Teil schweren Krankheitssymptomen zuverlässig zu schützen.

In diesem Zusammenhang ist zusätzlich noch die Tatsache zu berücksichtigen, daß der Vorrat an in aufgetautem und/oder erwärmtem Zustand bereitgehaltenem Infusionsoder Transfusionsgut umso kleiner gehalten werden kann, je schneller das Auftauen und/oder Erwärmen durchgeführt werden kann.

Es sind daher Auftau- und Erwärmgeräte der eingangs genannten Art bekanntgeworden (vgl. beispielsweise DE-OS 24 18 155), mit denen sich tiefgefroren oder kühl gelagertes Infusions- oder Transfusionsgut durch Absorption von Mikrowellenstrahlung auftauen läßt und/oder tiefgefroren oder kühl gelagertes Infusions- oder Transfusionsgut auf Infusionstemperatur erwärmt werden kann. Geräte dieses Typs sind mit Mikrowellengeneratoren ausgestattet. Während des Temperierprozesses besteht jedoch die Gefahr, daß sich innerhalb des Behältnisses infolge einer fehlerhaften Anordnung des Behältnisses Stellen zu hoher Temperatur bilden können. Derartige Übertemperierungen beim Auftauen und/oder Erwärmen mit derartigen Geräten führen zu einer Schädigung von Bestandteilen des Infusions- oder Transfusionsguts. Abgesehen hiervon sind Mikrowellengeneratoren in Anschaffung, Betrieb und Wartung aufwendig und kostspielig.

Des weiteren sind Auftau- und Erwärmgeräte der eingangs genannten Art bekanntgeworden (vgl. beispielsweise EP 0 432 591 A1 ), bei denen die Temperierung des Behältnisinhalts mittels Wärmeleitung erfolgt. Innerhalb derartiger Geräte wird Wasser erwärmt und in einem geschlossenen Kreislauf entweder in ein Wasserbecken oder in zwei Wasserkissen gepumpt. Die Behältnisse mit dem Infusions- oder Transfusionsgut werden in das Wasserbecken eingehängt oder zwischen den beiden Wasserkissen gebettet. In diesem Zusammenhang hat sich die Handhabung von Wasserbecken bzw. Wasserkissen jedoch als ausgesprochen problematisch erwiesen, da sich im Wasser mit hoher Wahrscheinlichkeit gesundheitsschädliche Keime bilden können, die durch Haarrisse in den Behältnissen in das Infusions- oder Transfusionsgut gelangen können. Abgesehen hiervon ist die Handhabung derartiger Geräte sehr aufwendig, zeitraubend und teuer, zumal derartige Geräte aufgrund ihrer großen Masse nur sehr bedingt mobil einsetzbar sind. Demzufolge entsteht häufig die Situation, daß beispielsweise in einem Krankenhaus mit mehreren Stationen nur wenige Stationen über ein derartiges Auftau- und Erwärmgerät verfügen, dieses dann jedoch nur sehr eingeschränkt auslasten können.

Aus der EP 0 615 763 A2 ist ein Aufwärm- und Auftaugerät für in Behältnissen aufbewahrte medizinische Infusions- und Transfusionslösungen bekannt, mit einer Haltevorrichtung, die die Behältnisse aufnimmt und während des Aufwärm- und Auftauvorganges in einer hin- und hergehenden Bewegung hält. An der Haltevorrichtung ist mindestens ein Temperatursensor angeordnet, der mit dem an der Haltevorrichtung befestigten Behältnis in Berührung kommt. Das Aufwärm- und Auftaugerät weist des weiteren eine Strahlungsquelle mit mindestens einem Infrarotstrahler auf, dessen Hauptstrahlungsbereich im Wellenlängenbereich zwischen 600 nm und 1800 nm liegt.

Die DE 195 03 350 C1 beschreibt eine Vorrichtung zum Temperieren von Infusionsbeuteln während eines Infusionsvorganges, die eine Heizeinrichtung zur gesteuerten Erwärmung oder Warmhaltung der Infusionsbeutel umfaßt, wobei die Vorrichtung eine U-förmig gebogene Platte aufweist, in die der Infusionsbeutel einlegbar ist.

Die DE 37 41 051 C1 offenbart eine Auftau- und Temperiervorrichtung für medizinische Produkte, insbesondere für tiefgefrorene Plasma- oder Blutkonserven, mit wenigstens zwei ein temperiertes Medium enthaltenden, beidseitig des zu temperierenden Produkts angeordneten Kunststoffbeuteln, wobei wenigstens ein periodisch auf wenigstens einen der Kunststoffbeutel einwirkendes Druckglied vorgesehen ist.

Eine Vorrichtung gemäß dem Oberbegriff des Patentanspruches 1 ist aus der DE 195 48 826 A1 bekannt. Die Druckschrift beschreibt ein elektrisches Schnellauftaugerät zum raschen Auftauen einer in einem Behältnis enthaltenen gefrorenen Flüssigkeit, beispielsweise einer Injektions- oder Infusionslösung, die neben anderen Baugruppen eine Heizeinrichtung zur Aufnahme des die Flüssigkeit enthaltenden Behälters und einen Linearantrieb, der den Behälter während des Auftauens in Bewegung hält, aufweist.

### Beschreibung

In der DE 30 47 784 A1 wird ein Verfahren und eine Vorrichtung gemäß dem Oberbegriff des Patentanspruches 1 offenbart, durch das eine in einem flachen Kunststoffbeutel eingefrorene wässrige Suspension oder Lösungen von lebender Zellsubstanz ( Gefriergut ) mittels einer Heizeinrichtung aufgetaut und gleichzeitig durch die Schwenkbewegung der Heizplatten dem Gefriergut eine den Wärmeübergang beim Schmelzen erhöhende Konvektion aufgeprägt wird.

Aus der WO 88 / 09186 ist ein Beutelerwärmgerät bekannt, bei dem zwei zu erwärmende Dialysebeutel in eine Heizeinrichtung mit zwei sich nach unten verjüngenden Metallmulden gelegt werden. Die zu erwärmenden Dialysebeutel werden somit an drei Seiten durch die Kontaktflächen der Heizeinrichtung umschlossen.

Angesichts der vorstehend dargelegten Nachteile und Unzulänglichkeiten liegt der vorliegenden Erfindung die Aufgabe zugrunde, eine Vorrichtung zum Auftauen und / oder zum Erwärmen von in einem Behältnis befindlichem medizinischem Infusions - oder Transfusionsgut bereitzustellen, welche Vorrichtung sich unter Beibehaltung des Vorteils des schnellen Auftauens und / oder Erwärmens vor allem dadurch auszeichnet, dass lokale oder generelle Übertemperierungen des aufzutauenden und / oder zu erwärmenden Infusions ― oder Transfusionsguts zuverlässig vermieden werden und mithin ein schonendes Auftauen und / oder Erwärmen des Infusions - oder Transfusionsguts gewährleistet ist.

Des weiteren zielt die vorliegende Erfindung darauf ab, ein modulares Temperiersystem bereitzustellen, bei dem das Prinzip der Trockentemperierung verwirklicht ist.

Diese Aufgaben werden durch eine Vorrichtung zum Auftauen und / oder Erwärmen von in einem Behältnis befindlichem Infusions ― oder Transfusionsgut gelöst, welche Vorrichtung mindestens eine Heizeinrichtung aufweist, auf der sich das Behältnis während des Auftauens und / oder Erwärmens befindet und die das Behältnis während des Auftauens und / oder des Erwärmens zumindest zeitweise in Bewegung hält, wobei mindestens eine Wärmequelle vorgesehen ist, die die Umluft innerhalb der Vorrichtung temperiert. Die temperierte Umluft wird dabei durch ein auf das Behältnis gerichtetes Gebläse in Richtung auf das Behältnis bewegt.

Durch diese Wärmequelle und das auf das Behältnis gerichtete Gebläse kann dem im Behältnis befindlichen medizinischen Infusions ― oder Transfusionsgut vorzugsweise unabhängig von der Heizeinrichtung, insbesondere unabhängig von deren Betriebszustand, Wärme zugeführt werden. Hierbei bietet es sich beispielsweise aus betriebstechnischen Gründen gleichwohl an, die Zuführung von Wärme durch die Wärmequelle in Abstimmung mit der Zuführung von Wärme durch die Heizeinrichtung erfolgen zu lassen, so dass in diesem Fall eine zweifache thermische Kupplung vorliegt. Das Vorsehen einer derartigen, gewissermaßen externen Wärmequelle zusätzlich zur und in Wirkungskombination mit der Heizeinrichtung kann gerade in unvorhersehbaren Notfallsituationen, in denen sehr schnell große Mengen von Infusions ― oder Transfusionsgut benötigt werden, von lebensrettender Bedeutung sein.

Das Behältnis, in dem sich das tiefgefrorene oder gekühlte Infusions- oder Transfusionsgut befindet, wird mithin zum Auftauen und/oder zum Erwärmen auf die Heizeinrichtung gelegt, wobei ein gleichmäßiger und kontinuierlicher Auftauund/oder Erwärmvorgang das tiefgefrorene oder gekühlte Infusions- oder Transfusionsgut in einen verwendbaren Zustand bringt. Hierbei hat es sich überraschenderweise gezeigt, daß dieser gleichmäßige und kontinuierliche Auftauund/oder Erwärmvorgang schnell und sicher vonstatten geht, was bei einer Operation oder in einem Notfall eine unabdingbare Voraussetzung für den ordnungsgemäßen, in nicht seltenen Fällen lebensrettenden Einsatz des Infusions- oder Transfusionsguts ist.

Grundsätzlich erfolgt die Wärmeübertragung zwischen der Heizeinrichtung und dem Behältnis und damit dem Infusions- oder Transfusionsgut im Behältnis gemäß dem Wärmetauscher-Prinzip. Da auf den Einsatz beispielsweise von Wasser verzichtet wird, ist das Prinzip der Trockentemperierung verwirklicht. Es sei in diesem Zusammenhang nur ergänzend angemerkt, daß die Wärmestrahlung, die von dem das Behältnis umgebenden Raum emittiert und von dem Behältnis und damit vom Infusions- oder Transfusionsgut im Behältnis absorbiert wird, im wesentlichen vernachlässigt werden kann.

Durch einen für den mit dem Stand der Technik vertrauten Fachmann nicht vorhersehbaren Wirkungszusammenhang können lokale oder generelle Übertemperierungen hierbei zuverlässig dadurch verhindert werden, daß das Behältnis während des Auftauens und/oder des Erwärmens zumindest zeitweise in Bewegung gehalten wird. Durch diese Bewegung und die dadurch verursachte Bewegung des Infusions- oder Transfusionsguts ist eine überaus wünschenswerte Durchmischung des Infusions- oder Transfusionsguts gewährleistet, so daß Überhitzungen, seien sie örtlicher Art oder das gesamte Behältnis betreffend, sicher vermieden werden.

Durch die vorliegende Erfindung sind auch Vorrichtungen zum Auftauen und/oder zum Erwärmen von sich in einem Behältnis befindlichem medizinischem Infusions- oder Transfusionsgut mitumfaßt, welche Vorrichtungen zwei oder mehr Heizeinrichtungen aufweisen. Da es sich bei den zum Einsatz gelangenden Behältnissen zumeist um flache Beutel beispielsweise aus Kunststoff handelt, die sich im wesentlichen aus zwei rechteckförmigen Seitenflächen zusammensetzen, ist beispielsweise auch eine Ausgestaltungsform der vorliegenden Erfindung denkbar, bei der das Behältnis gewissermaßen in Sandwich-Position zwischen zwei Heizeinrichtungen angeordnet ist, so daß jede der beiden Seitenflächen des Behältnisses auf einer Heizeinrichtung aufliegt und von dieser erwärmt wird. Hierdurch können beide Seiten des Behältnisses simultan aufgetaut und/oder erwärmt werden, was gerade in unvorhersehbaren Notfallsituationen, in denen sehr schnell große Mengen an Infusions- oder Transfusionsgut benötigt werden, zu einer signifikanten Beschleunigung des Auftauund/oder Erwärmvorgangs führen und demzufolge von eminenter, weil lebensrettender Bedeutung sein kann.

Gemäß einer besonders vorteilhaften Ausgestaltung der vorliegenden Erfindung weist die Heizeinrichtung im wesentlichen eine Heizplatte und eine Heizquelle auf. Hierbei liegt das Behältnis mit dem aufzutauenden und/oder zu erwärmenden Infusions- oder Transfusionsgut auf der Heizplatte, die durch die Heizquelle auf die gewünschte Temperatur erwärmt wird. Diese Temperatur wird dann im Laufe des Auftauund/oder Erwärmvorgangs abhängig von den jeweiligen Erfordernissen konstant gehalten oder nach unten bzw. oben angepaßt.

In diesem Zusammenhang sollte nicht unerwähnt bleiben, daß die jeweilige Temperatur des Behältnisses in etwa der jeweiligen Temperatur des an die Wand des Behältnisses angrenzenden Infusions- oder Transfusionsguts entspricht und mithin die maximale Temperatur oder sogenannte "Kontakttemperatur" des Infusions- oder Transfusionsguts darstellt. Die Größe dieser Kontakttemperatur richtet sich nach der Empfehlung "A. Hildebrand: Monographic, Human-Blutkonserve, Bekanntmachung über die Zulassung und Registrierung von Arzneimitteln" des Bundesgesundheitsamtes im Bundesanzeiger, Nr. 182, 1989, Seiten 4567 bis 4571, wobei sie jedoch jederzeit verändert werden kann. Derzeit wird eine Kontakttemperatur von 36°C eingestellt, wobei die Ausregelung der Größe der Kontakttemperatur beispielsweise in Abhängigkeit von der Temperatur der Heizplatte sowie in Abhängigkeit von der Durchschnittstemperatur des Infusions- oder Transfusionsguts erfolgt.

Da das Behältnis mit dem aufzutauenden und/oder zu erwärmenden Infusions- oder Transfusionsgut auf der Heizplatte liegt, ist es besonders vorteilhaft, wenn die Heizplatte eine Ausnehmung aufweist. Weist diese Ausnehmung hierbei zweckmäßigerweise die Form eines Teils des Behältnisses auf, so kann ein optimaler Wärmeübertrag von der Heizplatte auf das Behältnis und mithin auf das aufzutauende und/oder zu erwärmende Infusions- oder Transfusionsgut erfolgen, was den Vorgang des Auftauens und/oder Erwärmens erheblich schneller und zuverlässiger macht. Dieser Effekt tritt insbesondere dann zutage, wenn der genannte Teil eine Seitenfläche des Behältnisses ist, weil sich das gefüllte Behältnis dann in nahezu idealer Weise an die Form der Ausnehmung anschmiegt.

Gerade im Rahmen des vorgenannten Anpassungsprozesses in bezug auf die Temperatur erweist es sich in der praktischen Ausgestaltung der Heizeinrichtung als vorteilhaft, wenn die Heizquelle in der Heizplatte integriert ist. Auf diese Weise können die Wärmeverluste beispielsweise an die Umgebung minimal gehalten werden, was unter dem Aspekt der Kosteneinsparung und des Umweltschutzes als erfreulich zu bewerten ist.

Im Zusammenhang mit vorgenannter Temperaturanpassung kann die Vorrichtung gemäß der vorliegenden Erfindung technisch so ausgelegt werden, daß eben diese Temperatur der Heizeinrichtung stufenlos einstellbar ist. Dies ermöglicht eine besonders flexible, genaue und rasche Reaktion auf spezifische Notwendigkeiten, wie sie gerade bei Operationen und Notfalleinsätzen häufig auftreten können.

Wie vorstehend bereits ausgeführt, wird das Behältnis während des Auftauens und/oder des Erwärmens zumindest zeitweise in Bewegung gehalten. Hierfür erweist es sich in der konstruktiven Ausgestaltung der Erfindung als ausgesprochen zweckmäßig, eine Antriebseinrichtung zum Bewegen der Heizeinrichtung vorzusehen. Diese Antriebseinrichtung weist vorzugsweise eine elektromechanische Antriebseinheit auf, wobei diese elektromechanische Antriebseinheit beispielsweise in einem Befestigungssockel integriert sein kann. Des weiteren kann die elektromechanische Antriebseinheit eine Drehachse aufweisen.

Gemäß einer vorteilhaften Ausführungsform ist an der elektromechanischen Antriebseinheit eine Verbindung zur Heizeinrichtung angeordnet, durch welche Verbindung die durch die elektromechanische Antriebseinheit hervorgerufene Bewegung zur Heizeinrichtung übertragen werden kann, so daß die daraus resultierende Bewegung der Heizeinrichtung und mithin des Infusions- oder Transfusionsguts die erwünschte Durchmischung des Infusions- oder Transfusionsguts bewirkt. Zweckmäßigerweise ist die Verbindung hierbei als mechanische Steckverbindung ausgebildet.

Abhängig von der jeweiligen Konsistenz bzw. vom jeweiligen Aggregatzustand (fest oder flüssig) des Infusions- oder Transfusionsguts kann die Heizeinrichtung etwa eine hin- und hergehende Bewegung ausführen, wobei diese hin- und hergehende Bewegung beispielsweise eine Drehbewegung sein kann. Alternativ oder in Ergänzung hierzu kann die Heizeinrichtung aber auch eine Schwenkbewegung ausführen, wobei das wesentliche Kriterium für die Art und Geschwindigkeit der Bewegung die jeweilige konkrete Zusammensetzung des Infusions- oder Transfusionsguts sowie der jeweilige konkrete Zustand des Infusions- oder Transfusionsguts ist.

Gemäß einer vorteilhaften Ausführungsform der vorliegenden Erfindung ist mindestens ein Bewegungssensor, beispielsweise ein Neigungsschalter, vorgesehen, der die Bewegung der Heizvorrichtung und somit des Behältnisses mit dem darin befindlichen Infusions- oder Transfusionsgut erfaßt. Im Zuge dieser Erfassung ist es zweckmäßig, die in bezug auf die Bewegung aufgenommenen Daten geräteintern zu verarbeiten und der Benutzerin oder dem Benutzer in aufbereiteter Form zur Verfügung zu stellen, beispielsweise mittels mindestens einer Anzeigeeinheit, die dem Bewegungssensor zugeordnet sein kann. Hierdurch können der Benutzerin oder dem Benutzer die im Zusammenhang mit der Bewegung relevanten Informationen in stets aktualisierter Form zur Verfügung stehen.

Nach einer erfindungswesentlichen Weiterbildung der vorliegenden Vorrichtung ist mindestens eine Wärmequelle vorgesehen, die zweckmäßigerweise die Umluft innerhalb der Vorrichtung temperiert. Durch diese Wärmequelle kann dem sich im Behältnis befindlichen medizinischen Infusions- oder Transfusionsgut unabhängig von der Heizeinrichtung, insbesondere unabhängig von deren Betriebszustand, Wärme zugeführt werden. Hierbei bietet es sich beispielsweise aus betriebstechnischen Gründen gleichwohl an, die Zuführung von Wärme durch die Wärmequelle in Abstimmung mit der Zuführung von Wärme durch die Heizeinrichtung erfolgen zu lassen, so daß in diesem Falle eine zweifache thermische Kopplung vorliegt. Es liegt hierbei auf der Hand, daß das Vorsehen einer derartigen, gewissermaßen externen Wärmequelle zusätzlich zur und in Wirkungskombination mit der Heizeinrichtung gerade in unvorhersehbaren Notfallsituationen, in denen sehr schnell große Mengen an Infusions- oder Transfusionsgut benötigt werden, von eminenter, weil lebensrettender Bedeutung sein kann.

Für Verwendungsgebiete, bei denen eine besonders schnelle Durchführung des Auftauund/oder Erwärmvorgangs erforderlich ist, kann es zweckmäßig sein, zwei Wärmequellen vorzusehen. Diese zwei Wärmequellen können auf das Behältnis gerichtet und/oder in bezug auf die Heizeinrichtung symmetrisch angeordnet sein, um ein räumlich gleichmäßiges Auftauen und/oder Erwärmen des Infusions- oder Transfusionsguts innerhalb des Behältnisses zu gewährleisten.

Will man die vorliegende Vorrichtung hierbei besonders vorteilhaft ausgestalten, so ordnet man die Wärmequelle auf der der Heizeinrichtung abgewandten Seite des Behältnisses an, wobei die Wärmequelle üblicherweise, aber nicht notwendigerweise zum Behältnis beabstandet angeordnet sein wird. Damit ist ein Auftauen und/oder Erwärmen des sich im Behältnis befindlichen Infusions- oder Transfusionsguts von beiden Seiten möglich, wobei ergänzend anzumerken ist, daß es sich bei den zum Einsatz gelangenden Behältnissen zumeist um flache Beutel beispielsweise aus Kunststoff handelt, die sich im wesentlichen aus zwei rechteckförmigen Seitenflächen zusammensetzen. Während eine dieser Seitenflächen auf der Heizeinrichtung aufliegt und von dieser erwärmt wird, ist die andere Seitenfläche im Falle des vorstehend beschriebenen Anordnens der Wärmequelle auf der der Heizeinrichtung abgewandten Seite des Behältnisses der Konvektion und/oder Wärmeleitung durch eben diese Wärmequelle ausgesetzt, so daß beide Seiten des Behältnisses simultan aufgetaut und/oder erwärmt werden können.

Gemäß einer vorteilhaften Ausgestaltungsform der vorliegenden Erfindung ist dem Behältnis mindestens ein Strahlungs- und/oder Temperatursensor zugeordnet, wobei der Strahlungs- und/oder Temperatursensor vorzugsweise mindestens ein Oberflächensegment des Behältnisses erfaßt und es sich bei dem Strahlungs- und/oder Temperatursensor beispielsweise um einen Infrarotsensor oder ein Pyrometer handelt. Über den Strahlungs- und/oder Temperatursensor kann die Wärmestrahlung bzw. Temperatur des Infusions- oder Transfusionsguts im Behältnis ermittelt werden, wobei die von dem Oberflächensegment auf den Strahlungs- und/oder Temperatursensor abgeleitete Wärmestrahlung ein direktes Maß für die Temperatur des Infusions- oder Transfusionsguts ist.

Der Benutzerin oder dem Benutzer kann die entsprechende, für die Verwendung des Infusions- oder Transfusionsguts ausgesprochen relevante Information vorzugsweise mittels mindestens einer Anzeigeeinheit zur Verfügung gestellt werden, die dem Strahlungs- und/oder Temperatursensor zugeordnet sein kann.

Zweckmäßigerweise ist der Heizeinrichtung mindestens ein Strahlungs- und/oder Temperatursensor zugeordnet, durch den die jeweilige Temperatur der Heizeinrichtung schnell und zuverlässig ermittelt werden kann. Der Strahlungsund/oder Temperatursensor, bei dem es sich beispielsweise um ein elektrisches Thermoelement handelt, ist hierbei üblicherweise in die Heizeinrichtung, beispielsweise in die Heizplatte integriert.

Auch hierbei erweist es sich als vorteilhaft, dem Strahlungs- und/oder Temperatursensor mindestens eine Anzeigeeinheit zuzuordnen, die die Temperatur der Heizeinrichtung wiedergibt.

Alternativ oder in Ergänzung hierzu kann mindestens ein Strahlungs- und/oder Temperatursensor zur Erfassung der Umgebungsbedingungen innerhalb der Vorrichtung vorgesehen sein. Hierbei kann beispielsweise ein elektrisches Thermoelement zum Einsatz gelangen, wobei dem Strahlungs- und/oder Temperatursensor wiederum mindestens eine Anzeigeeinheit zugeordnet sein kann. Sinn und Zweck dieses Strahlungs- und/oder Temperatursensors ist die meßtechnische Registrierung der temperaturbezogenen Umgebungsbedingungen, insbesondere was die Umluft innerhalb der Vorrichtung gemäß der vorliegenden Erfindung anbelangt.

Durch das optionale Vorsehen der vorgenannten Strahlungs- und/oder Temperatursensoren kann sehr zuverlässig eine Regelung der Temperatur, insbesondere der maximalen Temperatur des Infusions- oder Transfusionsguts im Behältnis erzielt werden. Entscheidend ist hierbei unter anderem, daß anhand der zu Beginn und/oder im Laufe der Temperierung kontinuierlich gemessenen Oberflächentemperatur erkannt werden kann, in welchem Aggregatzustand (fest oder flüssig) sich das Infusions- oder Transfusionsgut im Behältnis befindet. Auch die meßtechnische Erfassung der jeweiligen Temperatur der Heizeinrichtung sowie der temperaturbezogenen Umgebungsbedingungen, insbesondere was die Umluft innerhalb der Vorrichtung gemäß der vorliegenden Erfindung anbelangt, ist von erheblicher Bedeutung für den Temperierprozeß:

Im schnellen Ansprechen auf die von den vorgenannten Strahlungs- und/oder Temperatursensoren bereitgestellten Informationen kann demzufolge ein automatischer Temperierprozeß aktiviert werden.

Des weiteren kann durch die vorgenannten Strahlungs- und/oder Temperatursensoren im (sehr unwahrscheinlichen) Falle einer Übertemperierung des Infusions- oder Transfusionsguts ohne jegliche zeitliche Verzögerung eine Fehlermeldung ausgegeben werden, um eine weitere Fehlfunktion und damit gegebenenfalls verbundenen Schaden zu verhindern.

Im Hinblick auf die temperaturbezogenen Umgebungsbedingungen, insbesondere auf die Umluft innerhalb der Vorrichtung gemäß der vorliegenden Erfindung kann sich auch eine Ausgestaltung als besonders vorteilhaft erweisen, bei der mindestens eine Belüftungseinrichtung vorgesehen ist. Diese Belüftungseinrichtung kann beispielsweise eine Ventilatoreinrichtung sein, wobei durch eine derartige Belüftungseinrichtung die Umluft bewegt und somit die Auftau- und/oder Erwärmzeit reduziert wird.

Vor diesem Hintergrund kann es sich als besonders sinnvoll erweisen, die Belüftungseinrichtung im Bereich zwischen der Heizeinrichtung und der Wärmequelle anzuordnen, um mittels der Belüftungseinrichtung die Umluft zu bewegen und für eine gleichmäßige Wärmezufuhr von der Wärmequelle zum aufzutauenden und/oder zu erwärmenden Infusions- oder Transfusionsgut zu sorgen.

Wie bereits weiter oben ausgeführt, kann es für Verwendungsgebiete, bei denen eine besonders schnelle Durchführung des Auftau- und/oder Erwärmvorgangs erforderlich ist, zweckmäßig sein, zwei Wärmequellen vorzusehen. Für diesen Fall können auch zwei Belüftungseinrichtungen vorgesehen sein, die wiederum auf das Behältnis gerichtet und/oder in bezug auf die Heizeinrichtung symmetrisch angeordnet sein können, um ein räumlich gleichmäßiges Auftauen und/oder Erwärmen des Infusionsoder Transfusionsguts innerhalb des Behältnisses zu gewährleisten.

Nach einer besonders erfinderischen Weiterbildung der vorliegenden Erfindung ist mindestens eine Kontrolleinrichtung zur Überwachung des Auftauens und/oder des Erwärmens vorgesehen. Hierzu werden in der Kontrolleinrichtung bestimmte relevante Systemvariablen berechnet und mit den realen meßbaren Größen verglichen, wodurch sich der Temperierprozeß auf Plausibilität überprüfen läßt.

Darüber hinaus sind mittels der Kontrolleinrichtung, beispielsweise auf der Grundlage der von den vorgenannten Strahlungs- und/oder Temperatursensoren bereitgestellten Informationen (Temperatur von Oberflächensegmenten des Behältnisses; Temperatur der Heizeinrichtung; temperaturbezogene Umgebungsbedingungen), auch Systemgrößen rekonstruierbar, die im Zusammenhang mit dem Temperierprozeß von wesentlicher Bedeutung sind, jedoch nicht unmittelbar erfaßt oder gemessen werden können, so etwa die Temperatur des Infusions- oder Transfusionsguts im Inneren des Behältnisses bzw. die entsprechende Temperaturverteilung. Die Kontrolleinrichtung fungiert hierbei mithin als ein Zustandsbeobachter, der die Berechnung und/oder das Nachvollziehen derartiger, nicht direkt ermittelbarer Systemgrößen ermöglicht.

Da nun mit der Vorrichtung gemäß der vorliegenden Erfindung Infusions- oder Transfusionsgut unterschiedlicher Konsistenz aufzutauen und/oder zu erwärmen ist und die entsprechenden Behältnisse unterschiedliche Beschaffenheiten, Füllmengen und Materialeigenschaften aufweisen, wird durch die Kontrolleinrichtung grundsätzlich eine Temperierung berechnet, bei der eine lokale Überhitzung am wahrscheinlichsten ist. Somit können unzulässige Temperaturen in jedem denkbaren Falle zuverlässig ausgeschlossen werden.

Nach einer weiteren, besonders erfinderischen Weiterbildung der vorliegenden Erfindung ist mindestens eine Regeleinrichtung zum Steuern des Auftauens und/oder des Erwärmens vorgesehen. Gemäß einer optionalen Ausgestaltungsform kann hierbei vorgesehen sein, daß die Kontrolleinrichtung und die Regeleinrichtung miteinander in Verbindung stehen, wobei in diesem Falle die Ausgangsgrößen der Kontrolleinrichtung und des realen Auftau- und/oder Erwärmvorgangs der Regeleinrichtung zugeführt werden und mithin ein gefahrloses und schnelles Temperieren vollständig automatisiert werden kann. In letzterem Falle, aber nicht nur in diesem, können die mindestens eine Kontrolleinrichtung und die mindestens eine Regeleinrichtung mit der Heizeinrichtung in Verbindung stehen.

Gemäß einer vorteilhaften Weiterbildung der vorliegenden Erfindung ist die Vorrichtung in einer Wärmekammer angeordnet. Dies erfüllt den Zweck, das Volumen der Raumluft zu begrenzen.

Nach einer bevorzugten Ausführungsform der Vorrichtung ist die Wärmekammer in ein Gehäuse integriert. Hierdurch ist es möglich, die Vorrichtung transportabel auszugestalten, so daß diese je nach Einsatzort und -zweck ohne großen Aufwand flexibel und mobil verwendet werden kann.

Zweckmäßigerweise ist das Gehäuse mit einer Klappe versehen, um der Benutzerin oder dem Benutzer einen leichten und ungehinderten Zugang zu den Gerätschaften innerhalb des Gehäuses zu ermöglichen. Optionalerweise kann dem Gehäuse und der Klappe hierbei ein Sensor zugeordnet sein, der beispielsweise als Mikroschalter ausgebildet ist und zweckmäßigerweise zur Erfassung der Schließposition der Klappe (geschlossen oder offen) vorgesehen ist.

Gemäß einer besonders erfinderischen Weiterbildung stellt jede Vorrichtung gemäß der vorliegenden Erfindung eine modulare Funktionseinheit dar, wobei jede modulare Funktionseinheit zweckmäßigerweise eigenständig arbeitet und wobei praktischerweise zwei oder mehr modulare Funktionseinheiten zusammensetzbar sind. Hierdurch können die Vorrichtungen gemäß der vorliegenden Erfindung als mobile Ausführungen geliefert werden, so daß die Möglichkeit besteht, eine bestimmte Anzahl von Vorrichtungen beispielsweise bei Massivinfusionen oder -transfusionen zentral oder verteilt, das heißt dezentral einzusetzen.

Auf diese Weise wird ein Temperiersystem bereitgestellt, das sich in Abhängigkeit von der Menge des aufzutauenden und/oder zu erwärmenden Infusions- oder Transfusionsguts und in Abhängigkeit von der Zeit, in der die Temperierung erfolgen soll, aus einer bestimmten Anzahl von Vorrichtungen zusammensetzt. Die einzelnen Vorrichtungen bilden hierbei die Module des Temperiersystems, so daß ein modulares Temperiersystem zur Verfügung gestellt wird, das jedoch nicht verkopplungsorientiert ist. Mittels dieser Struktur kann die Voraussetzung für eine optimale Auslastung der Vorrichtungen gemäß der vorliegenden Erfindung geschaffen werden.

Die Erfindung wird nachstehend anhand der in den Figuren 1 bis 3 schematisch dargestellten Ausführungsbeispiele exemplarisch näher erläutert. Es zeigen:
- Figur 1:: eine Vorrichtung zum Auftauen und/oder zum Erwärmen von sich in einem Behältnis befindlichem medizinischem Infusions- oder Transfusionsgut gemäß einem ersten Ausführungsbeispiel der vorliegenden Erfindung;
- Figur 2:: eine Vorrichtung zum Auftauen und/oder zum Erwärmen von sich in einem Behältnis befindlichem medizinischem Infusions- oder Transfusionsgut gemäß einem zweiten Ausführungsbeispiel der vorliegenden Erfindung; und
- Figur 3:: ein prinzipielles Schaltbild für eine Regelkreisstruktur für eine Vorrichtung zum Auftauen und/oder zum Erwärmen von sich in einem Behältnis befindlichem medizinischem Infusions- oder Transfusionsgut gemäß der vorliegenden Erfindung.

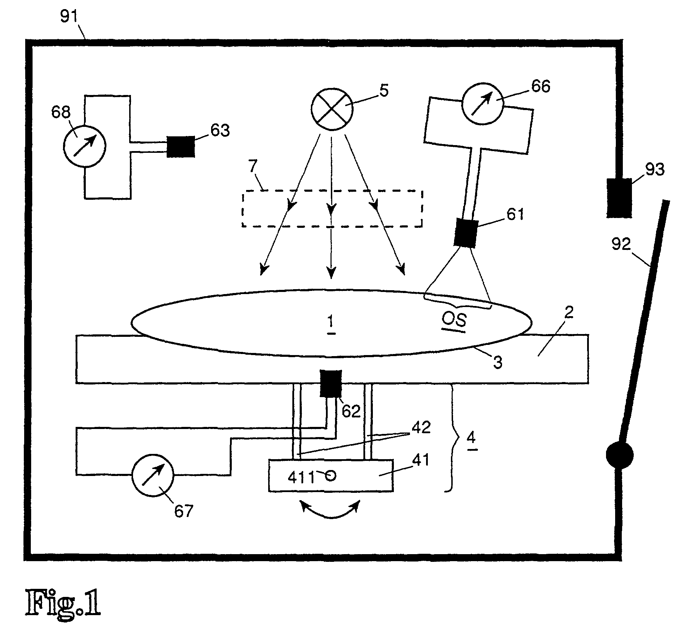

Gleiche oder ähnliche Teile sind in den Figuren 1 bis 3 mit den gleichen Bezugszeichen versehen.

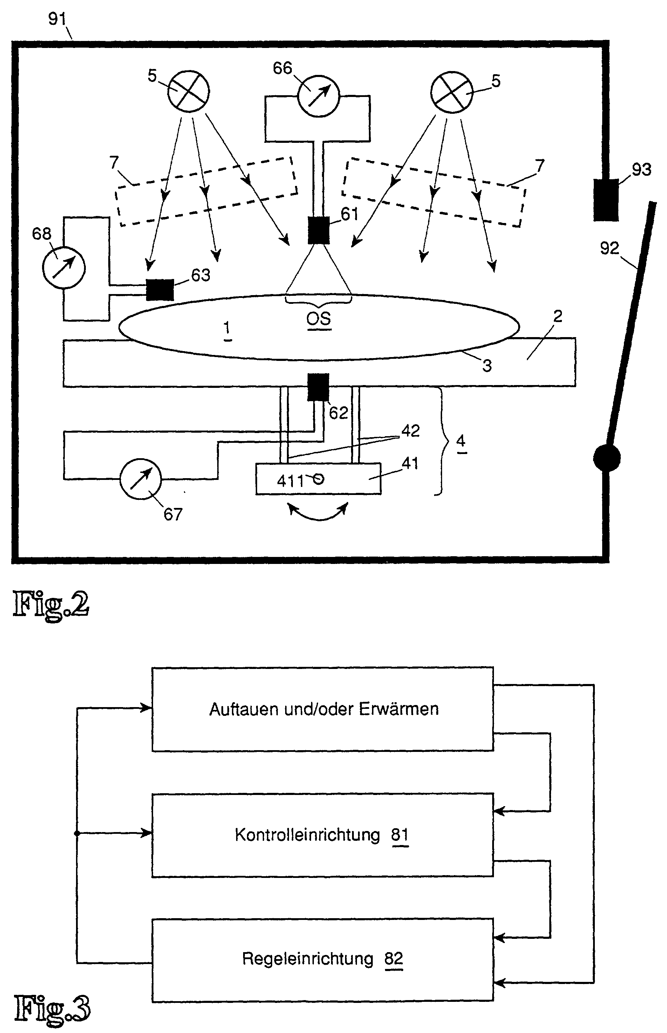

Die Figuren 1 und 2 zeigen zwei Ausführungsbeispiele einer Vorrichtung zum Auftauen und/oder zum Erwärmen von sich in einem Behältnis 1 befindlichem medizinischem Infusions- oder Transfusionsgut, beispielsweise eines Blutpräparats wie etwa einer Blutkonserve oder einer Blutplasmakonserve. Die Vorrichtung in den Figuren 1 und 2 weist jeweils eine Heizeinrichtung 2 auf, auf der sich das Behältnis 1 während des Auftauens und/oder des Erwärmens befindet und die das Behältnis 1 während des Auftauens und/oder des Erwärmens zumindest zeitweise in Bewegung hält.

Derartiges Infusions- oder Transfusionsgut wird im Regelfall im tiefgefrorenen oder gekühlten Zustand gelagert, so daß dessen Temperatur während der Aufbewahrung und Bereithaltung weit unterhalb der Körpertemperatur liegt. Um beispielsweise bei einer Operation oder in einem Notfall zur Verfügung zu stehen, muß das tiefgefrorene bzw. gekühlte Infusions- oder Transfusionsgut vor der Retransfusion schnell und gleichwohl schonend aufgetaut bzw. erwärmt werden, um die Empfängerin bzw. den Empfänger des Infusions- oder Transfusionsguts vor Unterkühlungen und den daraus resultierenden, zum Teil schweren Krankheitssymptomen zuverlässig zu schützen.

In diesem Zusammenhang ist zusätzlich noch die Tatsache zu berücksichtigen, daß der Vorrat an in aufgetautem und/oder erwärmtem Zustand bereitgehaltenem Infusionsoder Transfusionsgut umso kleiner gehalten werden kann, je schneller das Auftauen und/oder Erwärmen durchgeführt werden kann, was bei den zwei in den Figuren 1 und 2 dargestellten Ausführungsbeispielen bestens möglich ist:

Durch die vorliegende, in den Figuren 1 und 2 beispielhaft gezeigte Erfindung wird eine Vorrichtung bereitgestellt, die sich unter Beibehaltung des Vorteils des schnellen Auftauens und/oder Erwärmens vor allem dadurch auszeichnet, daß lokale oder generelle Übertemperierungen des aufzutauenden und/oder zu erwärmenden Infusions- oder Transfusionsguts zuverlässig vermieden werden und mithin ein schonendes Auftauen und/oder Erwärmen des Infusions- oder Transfusionsguts gewährleistet ist. Hierbei wird ein modulares Temperiersystem zur Verfügung gestellt, bei dem das Prinzip der Trockentemperierung verwirklicht ist.

Das Behältnis 1, in dem sich das tiefgefrorene oder gekühlte Infusions- oder Transfusionsgut befindet, wird zum Auftauen und/oder zum Erwärmen auf die Heizeinrichtung 2 gelegt, wobei ein gleichmäßiger und kontinuierlicher Auftauund/oder Erwärmvorgang das tiefgefrorene oder gekühlte Infusions- oder Transfusionsgut in einen verwendbaren Zustand bringt. Hierbei hat es sich gezeigt, daß dieser gleichmäßige und kontinuierliche Auftau- und/oder Erwärmvorgang schnell und sicher vonstatten geht, was bei einer Operation oder in einem Notfall eine unabdingbare Voraussetzung für den ordnungsgemäßen, in nicht seltenen Fällen lebensrettenden Einsatz des Infusions- oder Transfusionsguts ist.

Grundsätzlich erfolgt die Wärmeübertragung zwischen der Heizeinrichtung 2 und dem Behältnis 1 und damit dem Infusions- oder Transfusionsgut im Behältnis 1 gemäß dem Wärmetauscher-Prinzip. Da auf den Einsatz beispielsweise von Wasser verzichtet wird, ist das Prinzip der Trockentemperierung verwirklicht. Es sei in diesem Zusammenhang nur ergänzend angemerkt, daß die Wärmestrahlung, die von dem das Behältnis 1 umgebenden Raum emittiert und von dem Behältnis 1 und damit vom Infusions- oder Transfusionsgut im Behältnis 1 absorbiert wird, im wesentlichen vernachlässigt werden kann.

Lokale oder generelle Übertemperierungen können hierbei zuverlässig dadurch verhindert werden, daß das Behältnis 1 während des Auftauens und/oder des Erwärmens zumindest zeitweise in Bewegung gehalten wird. Durch diese Bewegung und die dadurch verursachte Bewegung des Infusions- oder Transfusionsguts ist eine überaus wünschenswerte Durchmischung des Infusions- oder Transfusionsguts gewährleistet, so daß Überhitzungen, seien sie örtlicher Art oder das gesamte Behältnis 1 betreffend, sicher vermieden werden.

In den Ausführungsbeispielen der Figuren 1 und 2 weist die Heizeinrichtung 2 eine Heizplatte und eine Heizquelle auf. Letztere ist nicht explizit dargestellt, weil die Heizquelle in der Heizplatte integriert ist. Auf diese Weise können die Wärmeverluste beispielsweise an die Umgebung minimal gehalten werden, was unter dem Aspekt der Kosteneinsparung und des Umweltschutzes als erfreulich zu bewerten ist.

Hierbei liegt das Behältnis 1 mit dem aufzutauenden und/oder zu erwärmenden Infusions- oder Transfusionsgut auf der Heizplatte, die durch die Heizquelle auf die gewünschte Temperatur erwärmt wird. Diese Temperatur wird dann im Laufe des Auftau- und/oder Erwärmvorgangs abhängig von den jeweiligen Erfordernissen konstant gehalten oder nach unten bzw. oben angepaßt.

In diesem Zusammenhang sollte nicht unerwähnt bleiben, daß die jeweilige Temperatur des Behältnisses 1 in etwa der jeweiligen Temperatur des an die Wand des Behältnisses 1 angrenzenden Infusions- oder Transfusionsguts entspricht und mithin die maximale Temperatur oder sogenannte "Kontakttemperatur" des Infusions- oder Transfusionsguts darstellt. Die Größe dieser Kontakttemperatur richtet sich nach der Empfehlung "A. Hildebrand: Monographic, Human-Blutkonserve, Bekanntmachung über die Zulassung und Registrierung von Arzneimitteln" des Bundesgesundheitsamtes im Bundesanzeiger, Nr. 182, 1989, Seiten 4567 bis 4571, wobei sie jedoch jederzeit verändert werden kann. Derzeit wird eine Kontakttemperatur von 36°C eingestellt, wobei die Ausregelung der Größe der Kontakttemperatur beispielsweise in Abhängigkeit von der Temperatur der Heizplatte sowie in Abhängigkeit von der Durchschnittstemperatur des Infusions- oder Transfusionsguts erfolgt.

Da das Behältnis 1 mit dem aufzutauenden und/oder zu erwärmenden Infusions- oder Transfusionsgut auf der Heizplatte liegt, weist die Heizplatte bei den in den Figuren 1 und 2 gezeigten Ausführungsbeispielen eine Ausnehmung 3 auf. Da diese Ausnehmung 3 hierbei die Form eines Teils des Behältnisses 1 aufweist, kann ein optimaler Wärmeübertrag von der Heizplatte auf das Behältnis 1 und mithin auf das aufzutauende und/oder zu erwärmende Infusions- oder Transfusionsgut erfolgen, was den Vorgang des Auftauens und/oder Erwärmens erheblich schneller und zuverlässiger macht. Dieser Effekt tritt insbesondere deshalb zutage, weil der genannte Teil bei den in den Figuren 1 und 2 dargestellten Ausführungsbeispielen eine Seitenfläche des Behältnisses 1 ist, so daß sich das gefüllte Behältnis 1 in nahezu idealer Weise an die Form der Ausnehmung 3 anschmiegt.

Im Zusammenhang mit vorgenannter Temperaturanpassung kann die Vorrichtung der Figuren 1 und 2 technisch so ausgelegt werden, daß eben diese Temperatur der Heizeinrichtung 2 stufenlos einstellbar ist. Dies ermöglicht eine besonders flexible, genaue und rasche Reaktion auf spezifische Notwendigkeiten, wie sie gerade bei Operationen und Notfalleinsätzen häufig auftreten können.

Wie vorstehend bereits ausgeführt, wird das Behältnis 1 während des Auftauens und/oder des Erwärmens zumindest zeitweise in Bewegung gehalten. Hierfür ist in der exemplarischen konstruktiven Ausgestaltung der Figuren 1 und 2 eine Antriebseinrichtung 4 zum Bewegen der Heizeinrichtung 2 vorgesehen. Diese Antriebseinrichtung 4 ist in Form einer elektromechanischen Antriebseinheit 41 realisiert, wobei diese elektromechanische Antriebseinheit 41 in den Ausführungsbeispielen der Figuren 1 und 2 in einem Befestigungssockel integriert ist und eine Drehachse 411 aufweist.

An der elektromechanischen Antriebseinheit 41 ist eine Verbindung 42 zur Heizeinrichtung 2 angeordnet, durch welche Verbindung 42 die durch die elektromechanische Antriebseinheit 41 hervorgerufene Bewegung zur Heizeinrichtung 2 übertragen wird, so daß die daraus resultierende Bewegung der Heizeinrichtung 2 und mithin des Infusions- oder Transfusionsguts die erwünschte Durchmischung des Infusions- oder Transfusionsguts bewirkt. In den Figuren 1 und 2 ist die Verbindung 42 hierbei als mechanische Steckverbindung ausgebildet.

Abhängig von der jeweiligen Konsistenz bzw. vom jeweiligen Aggregatzustand (fest oder flüssig) des Infusions- oder Transfusionsguts führt die Heizeinrichtung 2 eine hin- und hergehende Bewegung aus, wobei diese hin- und hergehende Bewegung beispielsweise eine Drehbewegung ist. Alternativ oder in Ergänzung hierzu führt die Heizeinrichtung 2 aber auch eine Schwenkbewegung aus, wobei das wesentliche Kriterium für die Art und Geschwindigkeit der Bewegung die jeweilige konkrete Zusammensetzung des Infusions- oder Transfusionsguts sowie der jeweilige konkrete Zustand des Infusions- oder Transfusionsguts ist.

Zwar ist dies bei den zwei in den Figuren 1 und 2 gezeigten Ausführungsbeispielen nicht explizit dargestellt, jedoch können ein oder mehr Bewegungssensoren, beispielsweise Neigungsschalter, vorgesehen sein, die die Bewegung der Heizvorrichtung 2 und somit des Behältnisses 1 mit dem darin befindlichen Infusionsoder Transfusionsgut erfassen. Im Zuge dieser Erfassung werden die in bezug auf die Bewegung aufgenommenen Daten geräteintern verarbeitet und der Benutzerin oder dem Benutzer in aufbereiteter Form zur Verfügung gestellt, beispielsweise mittels einer oder mehrerer Anzeigeeinheiten, die dem/n Bewegungssensor(en) zugeordnet sein können. Hierdurch können der Benutzerin oder dem Benutzer die im Zusammenhang mit der Bewegung relevanten Informationen in stets aktualisierter Form zur Verfügung stehen.

Im Ausführungsbeispiel der Figur 1 ist eine, im Ausführungsbeispiel der Figur 2 sind zwei Wärmequellen 5 vorgesehen, die die Umluft temperieren. Durch diese Wärmequellen 5 kann dem sich im Behältnis 1 befindlichen medizinischen Infusionsoder Transfusionsgut unabhängig von der Heizeinrichtung 2, insbesondere unabhängig von deren Betriebszustand, Wärme zugeführt werden. Hierbei bietet es sich beispielsweise aus betriebstechnischen Gründen gleichwohl an, die Zuführung von Wärme durch die Wärmequellen 5 in Abstimmung mit der Zuführung von Wärme durch die Heizeinrichtung 2 erfolgen zu lassen, so daß in diesem Falle eine zweifache thermische Kopplung vorliegt. Es liegt hierbei auf der Hand, daß das Vorsehen derartiger, gewissermaßen externer Wärmequellen 5 zusätzlich zur und in Wirkungskombination mit der Heizeinrichtung 2 gerade in unvorhersehbaren Notfallsituationen, in denen sehr schnell große Mengen an Infusions- oder Transfusionsgut benötigt werden, von eminenter, weil lebensrettender Bedeutung sein kann.

Gerade für Verwendungsgebiete, bei denen eine besonders schnelle Durchführung des Auftau- und/oder Erwärmvorgangs erforderlich ist, kann es hierbei zweckmäßig sein, zwei (vgl. Figur 2) oder mehr Wärmequellen 5 vorzusehen. Diese zwei Wärmequellen 5 sind in Figur 2 auf das Behältnis 1 gerichtet und in bezug auf die Heizeinrichtung 2 symmetrisch angeordnet, um ein räumlich gleichmäßiges Auftauen und/oder Erwärmen des Infusions- oder Transfusionsguts innerhalb des Behältnisses 1 zu gewährleisten.

In den Ausführungsbeispielen der Figuren 1 und 2 sind die Wärmequellen 5 auf der der Heizeinrichtung 2 abgewandten Seite des Behältnisses 1 angeordnet, wobei die Wärmequellen 5 zum Behältnis 1 beabstandet angeordnet sind. Damit ist ein Auftauen und/oder Erwärmen des sich im Behältnis 1 befindlichen Infusions- oder Transfusionsguts von beiden Seiten möglich, wobei ergänzend anzumerken ist, daß es sich bei den zum Einsatz gelangenden Behältnissen in den Ausführungsbeispielen der Figuren 1 und 2 um flache Beutel beispielsweise aus Kunststoff handelt. Während eine dieser Seitenflächen auf der Heizeinrichtung 2 aufliegt und von dieser erwärmt wird, ist die andere Seitenfläche der Konvektion und/oder Wärmeleitung durch diese Wärmequellen 5 ausgesetzt, so daß beide Seiten des Behältnisses 1 simultan aufgetaut und/oder erwärmt werden können.

In den Ausführungsbeispielen der Figuren 1 und 2 ist dem Behältnis jeweils ein Strahlungs- und/oder Temperatursensor 61 zugeordnet, der ein Oberflächensegment OS des Behältnisses 1 erfaßt. Bei dem Strahlungs- und/oder Temperatursensor 61 handelt es sich um einen Infrarotsensor oder ein Pyrometer. Über den Strahlungsund/oder Temperatursensor 61 kann die Wärmestrahlung bzw. Temperatur des Infusions- oder Transfusionsguts im Behältnis 1 ermittelt werden, wobei die von dem Oberflächensegment OS auf den Strahlungs- und/oder Temperatursensor 61 abgeleitete Wärmestrahlung ein direktes Maß für die Temperatur des Infusions- oder Transfusionsguts ist.

Der Benutzerin oder dem Benutzer kann die entsprechende, für die Verwendung des Infusions- oder Transfusionsguts ausgesprochen relevante Information mittels einer Anzeigeeinheit 66 zur Verfügung gestellt werden, die dem Strahlungs- und/oder Temperatursensor 61 zugeordnet sein kann.

In den Ausführungsbeispielen der Figuren 1 und 2 ist der Heizeinrichtung 2 jeweils ein Strahlungs- und/oder Temperatursensor 62 zugeordnet, durch den die jeweilige Temperatur der Heizeinrichtung 2 schnell und zuverlässig ermittelt werden kann. Der Strahlungs- und/oder Temperatursensor 62, bei dem es sich um ein elektrisches Thermoelement handelt, ist hierbei in die Heizeinrichtung 2, genauer gesagt in deren Heizplatte integriert.

Auch hier kann dem Strahlungs- und/oder Temperatursensor 62 eine Anzeigeeinheit 67 zugeordnet sein, die die Temperatur der Heizeinrichtung 2 wiedergibt.

In den Ausführungsbeispielen der Figuren 1 und 2 ist des weiteren jeweils ein Strahlungs- und/oder Temperatursensor 63 zur Erfassung der Umgebungsbedingungen innerhalb der Vorrichtung vorgesehen. Hierbei gelangt ein elektrisches Thermoelement zum Einsatz, wobei dem Strahlungs- und/oder Temperatursensor 63 wiederum eine Anzeigeeinheit 68 zugeordnet sein kann. Sinn und Zweck dieses Strahlungs- und/oder Temperatursensors 63 ist die meßtechnische Registrierung der temperaturbezogenen Umgebungsbedingungen, insbesondere was die Umluft innerhalb der in den Figuren 1 und 2 gezeigten Vorrichtungen anbelangt.

Durch das Vorsehen der Strahlungs- und/oder Temperatursensoren 61, 62, 63 kann sehr zuverlässig eine Regelung der Temperatur, insbesondere der maximalen Temperatur des Infusions- oder Transfusionsguts im Behältnis 1 erzielt werden. Entscheidend ist hierbei unter anderem, daß anhand der zu Beginn und/oder im Laufe der Temperierung kontinuierlich gemessenen Oberflächentemperatur erkannt werden kann, in welchem Aggregatzustand (fest oder flüssig) sich das Infusions- oder Transfusionsgut im Behältnis 1 befindet. Auch die meßtechnische Erfassung der jeweiligen Temperatur der Heizeinrichtung 2 sowie der temperaturbezogenen Umgebungsbedingungen, insbesondere was die Umluft innerhalb der Vorrichtung gemäß der vorliegenden Erfindung anbelangt, ist von erheblicher Bedeutung für den Temperierprozeß:

Im schnellen Ansprechen auf die von den Strahlungs- und/oder Temperatursensoren 61, 62, 63 bereitgestellten Informationen kann demzufolge ein automatischer Temperierprozeß aktiviert werden.

Des weiteren kann durch die Strahlungs- und/oder Temperatursensoren 61, 62, 63 im (sehr unwahrscheinlichen) Falle einer Übertemperierung des Infusions- oder Transfusionsguts ohne jegliche zeitliche Verzögerung eine Fehlermeldung ausgegeben werden, um eine weitere Fehlfunktion und damit gegebenenfalls verbundenen Schaden zu verhindern.

Im Hinblick auf die temperaturbezogenen Umgebungsbedingungen, insbesondere auf die Umluft innerhalb der Vorrichtung gemäß der vorliegenden Erfindung erweist es sich als besonders vorteilhaft, daß bei dem Ausführungsbeispiel in Figur 1 eine, bei dem Ausführungsbeispiel in Figur 2 zwei Belüftungseinrichtungen 7 vorgesehen sind. Diese Belüftungseinrichtungen 7 sind Ventilatoreinrichtungen, wobei durch derartige Belüftungseinrichtungen 7 die Umluft bewegt und somit die Auftau- und/oder Erwärmzeit reduziert wird.

Vor diesem Hintergrund ist es, wie in den Figuren 1 und 2 dargestellt, sinnvoll, die Belüftungseinrichtungen 7 im Bereich zwischen der Heizeinrichtung 2 und der Wärmequelle 5 anzuordnen, um mittels der Belüftungseinrichtung 7 die Umluft zu bewegen und für eine gleichmäßige Wärmezufuhr von der Wärmequelle 5 zum aufzutauenden und/oder zu erwärmenden Infusions- oder Transfusionsgut zu sorgen.

Wie bereits weiter oben ausgeführt, kann es für Verwendungsgebiete, bei denen eine besonders schnelle Durchführung des Auftau- und/oder Erwärmvorgangs erforderlich ist, zweckmäßig sein, zwei Wärmequellen 5 vorzusehen, so wie dies bei dem Ausführungsbeispiel in Figur 2 gezeigt ist. Für diesen Fall sind auch zwei Belüftungseinrichtungen 7 vorgesehen, die wiederum auf das Behältnis 1 gerichtet und in bezug auf die Heizeinrichtung 2 symmetrisch angeordnet sind, um ein räumlich gleichmäßiges Auftauen und/oder Erwärmen des Infusions- oder Transfusionsguts innerhalb des Behältnisses 1 zu gewährleisten.

Wie den Figuren 1 und 2 zu entnehmen ist, ist die Vorrichtung in einer Wärmekammer angeordnet. Dies erfüllt den Zweck, das Volumen der Raumluft zu begrenzen. Die Wärmekammer ist in ein Gehäuse 91 integriert, wodurch es möglich ist, die Vorrichtung transportabel auszugestalten, so daß diese je nach Einsatzort und - zweck ohne großen Aufwand flexibel und mobil verwendet werden kann.

In den Ausführungsbeispielen der Figuren 1 und 2 ist das Gehäuse 91 mit einer Klappe 92 versehen, um der Benutzerin oder dem Benutzer einen leichten und ungehinderten Zugang zu den Gerätschaften innerhalb des Gehäuses 91 zu ermöglichen. Dem Gehäuse 91 und der Klappe 92 ist hierbei ein Sensor 93 zugeordnet, der in den Ausführungsbeispielen der Figuren 1 und 2 als Mikroschalter ausgebildet ist und zur Erfassung der Schließposition der Klappe 92 (geschlossen oder offen) vorgesehen ist.

Obgleich dies in den vorstehend beschriebenen Figuren nicht explizit dargestellt ist, sind durch die vorliegende Erfindung auch Vorrichtungen zum Auftauen und/oder zum Erwärmen von sich in einem Behältnis 1 befindlichem medizinischem Infusions- oder Transfusionsgut mitumfaßt, welche Vorrichtungen zwei oder mehr Heizeinrichtungen 2 aufweisen. Da es sich bei den zum Einsatz gelangenden Behältnissen 1 zumeist um flache Beutel beispielsweise aus Kunststoff handelt, die sich im wesentlichen aus zwei rechteckförmigen Seitenflächen zusammensetzen, ist beispielsweise auch eine Ausgestaltungsform der vorliegenden Erfindung denkbar, bei der das Behältnis 1 gewissermaßen in Sandwich-Position zwischen zwei Heizeinrichtungen 2 angeordnet ist, so daß jede der beiden Seitenflächen des Behältnisses 1 auf einer Heizeinrichtung 2 aufliegt und von dieser erwärmt wird. Hierdurch können beide Seiten des Behältnisses 1 simultan aufgetaut und/oder erwärmt werden, was gerade in unvorhersehbaren Notfallsituationen, in denen sehr schnell große Mengen an Infusions- oder Transfusionsgut benötigt werden, zu einer signifikanten Beschleunigung des Auftau- und/oder Erwärmvorgangs führen und demzufolge von eminenter, weil lebensrettender Bedeutung sein kann.

Abschließend zeigt Figur 3 ein prinzipielles Schaltbild für eine Regelkreisstruktur für eine Vorrichtung zum Auftauen und/oder zum Erwärmen von sich in einem Behältnis 1 befindlichem medizinischem Infusions- oder Transfusionsgut gemäß der vorliegenden Erfindung. Im Rahmen der in den Figuren 1 und 2 dargestellten Ausführungsbeispiele der vorliegenden Erfindung ist hierzu eine Kontrolleinrichtung 81 zur Überwachung des Auftauens und/oder des Erwärmens vorgesehen. Hierbei werden in der Kontrolleinrichtung 81 bestimmte relevante Systemvariablen berechnet und mit den realen meßbaren Größen verglichen, wodurch sich der Temperierprozeß auf Plausibilität überprüfen läßt.

Darüber hinaus sind mittels der Kontrolleinrichtung 81, beispielsweise auf der Grundlage der von den Strahlungs- und/oder Temperatursensoren 61, 62, 63 bereitgestellten Informationen (Temperatur von Oberflächensegmenten OS des Behältnisses 1; Temperatur der Heizeinrichtung 2; temperaturbezogene Umgebungsbedingungen), auch Systemgrößen rekonstruierbar, die im Zusammenhang mit dem Temperierprozeß von wesentlicher Bedeutung sind, jedoch nicht unmittelbar erfaßt oder gemessen werden können, so etwa die Temperatur des Infusions- oder Transfusionsguts im Inneren des Behältnisses 1 bzw. die entsprechende Temperaturverteilung. Die Kontrolleinrichtung 81 fungiert hierbei mithin als ein Zustandsbeobachter, der die Berechnung und/oder das Nachvollziehen derartiger, nicht direkt ermittelbarer Systemgrößen ermöglicht.

Da nun mit der Vorrichtung gemäß der vorliegenden Erfindung Infusions- oder Transfusionsgut unterschiedlicher Konsistenz aufzutauen und/oder zu erwärmen ist und die entsprechenden Behältnisse 1 unterschiedliche Beschaffenheiten, Füllmengen und Materialeigenschaften aufweisen, wird durch die Kontrolleinrichtung 81 grundsätzlich eine Temperierung berechnet, bei der eine lokale Überhitzung am wahrscheinlichsten ist. Somit können unzulässige Temperaturen in jedem denkbaren Falle zuverlässig ausgeschlossen werden.

Des weiteren ist in Figur 3 eine Regeleinrichtung 82 zum Steuern des Auftauens und/oder des Erwärmens vorgesehen, wobei die Kontrolleinrichtung 81 und die Regeleinrichtung 82 miteinander in Verbindung stehen, so daß die Ausgangsgrößen der Kontrolleinrichtung 81 und des realen Auftau- und/oder Erwärmvorgangs der Regeleinrichtung 82 zugeführt werden und mithin ein gefahrloses und schnelles Temperieren vollständig automatisiert werden kann. Es versteht sich von selbst, daß hierzu die Kontrolleinrichtung 81 und die Regeleinrichtung 82 mit der Heizeinrichtung 2 in Verbindung stehen.

Jede Vorrichtung, wie sie in den Figuren 1 bis 3 exemplarisch dargelegt ist, stellt eine eigenständig arbeitende modulare Funktionseinheit dar, wobei im praktischen Einsatz zwei oder mehr modulare Funktionseinheiten zusammensetzbar sind. Hierdurch sind die Vorrichtungen gemäß der vorliegenden Erfindung als mobile Ausführungen lieferbar, so daß die Möglichkeit besteht, eine bestimmte Anzahl von Vorrichtungen beispielsweise bei Massivinfusionen oder -transfusionen zentral oder verteilt, das heißt dezentral einzusetzen.

Auf diese Weise wird ein Temperiersystem bereitgestellt, das sich in Abhängigkeit von der Menge des aufzutauenden und/oder zu erwärmenden Infusions- oder Transfusionsguts und in Abhängigkeit von der Zeit, in der die Temperierung erfolgen soll, aus einer bestimmten Anzahl von Vorrichtungen zusammensetzt. Die einzelnen Vorrichtungen bilden hierbei die Module des Temperiersystems, so daß ein modulares Temperiersystem zur Verfügung gestellt wird, das jedoch nicht verkopplungsorientiert ist. Mittels dieser Struktur kann die Voraussetzung für eine optimale Auslastung der Vorrichtungen gemäß der vorliegenden Erfindung geschaffen werden.

## Patentansprüche

1. Vorrichtung zum Auftauen und / oder zum Erwärmen von in einem Behältnis (1) befindlichen medizinischem Infusions- oder Transfusionsgut, welche Vorrichtung mindestens eine Heizeinrichtung (2) aufweist, auf der sich das Behältnis (1) während des Auftauens und / oder des Erwärmens befindet und die das Behältnis (1) während des Auftauens und / oder des Erwärmens zumindest zeitweise in Bewegung hält,
**dadurch gekennzeichnet,**
**daß** mindestens eine Wärmequelle (5) vorgesehen ist, die die Umluft innerhalb der Vorrichtung temperiert und mindestens ein auf das Behältnis gerichtetes Gebläse (7) vorgesehen ist, das die Umluft in Richtung auf das Behältnis (1) bewegt,
**daß** die Heizeinrichtung (2) im wesentlichen eine Heizplatte mit integrierter Heizquelle aufweist und daß die Temperatur der Heizeinrichtung (2) stufenlos einstellbar ist
und **daß** die Wärmequelle (5) auf der der Heizeinrichtung (2) abgewandten Seite des Behältnisses (1) im Abstand zu diesem angeordnet ist und daß die Wärmequelle (5) dem im Behältnis (1) befindlichen medizinischen Infusions- und Transfusionsgut unabhängig von der Heizeinrichtung (5) Wärme zuführt.

## Claims

1. Device for thawing and/or heating medical infusion or transfusion material located in a container (1), whereas the device has at least one heating device (2) on which the container (1) is located during thawing and/or heating and which keeps the container in movement, at least part of the time, during thawing and/or heating,
**marked by the fact that**
at least one heat source (5) is provided, which heats that circulated air inside the device and at least one fan (7) is provided that is pointed at the container, which moves the circulated air toward the container (1),
and that the heating device (2) primarily consists of a heating element with integrated heat source and that the temperature of the heating device (2) is infinitely adjustable
and that the heat source (5) on the side of the container (1) facing away from the heating device (2) is located at a distance and that the heat source (5) heats the medical infusion and transfusion material located in the container (1) independently from the heating device (5).

## Revendications

1. Dispositif destiné à décongeler et/ou réchauffer de produits médicaux d'infusion ou de transfusion contenus dans un récipient (1), lequel dispositif présente au moins un dispositif de chauffage (2) sur lequel le récipient (1) se trouve pendant la décongélation et/ou le réchauffage et maintient le récipient (1) au moins temporairement en mouvement pendant la décongélation et/ou le réchauffage,
**caractérisé par le fait,**
**qu'**au moins une source de chaleur (5) est prévue pour tempérer l'air en circulation au sein du dispositif et qu'une soufflerie (7) dirigée sur le récipient est prévue, qui met l'air de circulation en mouvement sur le récipient (1),
**que** le dispositif de chauffage (2) présente dans l'essentiel une plaque chauffante avec source de chaleur intégrée et que la température du dispositif de chauffage (2) est réglable en continu,
et **que** la source de chaleur (5) est disposée sur le côté du dispositif de chauffage opposé du récipient (1) à une certaine distance de ce demier et que la source de chaleur (5) approvisionne en chaleur les produits médicaux d'infusion ou de transfusion contenus dans un récipient (1) indépendamment du dispositif de chauffage (5).
